# EUROPEAN PATENT APPLICATION

(11) **EP 2 333 587 A1**
(43) Date of publication of application: **15.06.2011**
(21) Application number: 09450237.4
(22) Date of filing: 14.12.2009
(51) Int. Cl.: G01V 3/10, A61B 5/05

(54) **Device and method for magnetic induction tomography**

(71) Applicant: Technische Universität Graz, 8010 Graz (AT); Forschungsholding TU Graz GmbH, 8010 Graz (AT)
(72) Inventor: Scharfetter, Hermann, 8045 Graz (AT)
(74) Representative: Patentanwaltskanzlei Matschnig & Forsthuber OG

(57) **Abstract**

An_apparatus for magnetic induction tomography comprising at least one transmitter coil (LTX) for the introduction of an alternating magnetic field into a target body (OBJ) to be investigated, at least one receiver coil (LRX) for the pick-up of received signals and means for processing the received signals which reconstructs an image of the spatial electrical properties in the object from the amplitudes and phases of the received signals, the at least one transmitter coil being driven by a power amplifier (PA) having a low output impedance and working at two or more different frequencies (f_{L}, f_{H}), whereby two or more feeding paths (Pf_{L}, Pf_{H}) for the two or more frequencies (f_{L}, f_{H}) are provided for each transmitter coil (L_{TX}), in each feeding path the transmitter coil being driven at the corresponding frequency (f_{L}, f_{H}), via a λ/4-transformer (Tf_{L}, Tf_{H}), said coil being in series resonance with a series capacitor (C_{L}, C_{H}) of said path at said one frequency, whereas the other feeding path(s) showing essentially high impedance for said one frequency.

## Description

### Field of the invention and description of prior art

The present invention relates to an apparatus for magnetic induction tomography comprising at least two transmitter coils for the introduction of an alternating magnetic field into a target body to be investigated, at least one receiver coil for the pick-up of received signals and means for processing the received signals which reconstructs an image of the spatial electrical properties in the object from the amplitudes and phases of the received signals, each transmitter coil being driven by a power amplifier having a low output impedance and working at two or more different frequencies.

Magnetic induction tomography (MIT) is an imaging modality which, very much like electrical impedance tomography (EIT), aims at the mapping of the complex electrical conductivity κ = σ + jωεᵣε₀ inside an object, e. g. a human body. ω, σ and ε are the angular frequency, electrical conductivity and permittivity, respectively. MIT is therefore closely related to electrical bioimpedance. In contrast to EIT, however, the interrogating currents are not injected via electrodes but instead induced by alternating magnetic fields. Furthermore the received quantity is not the electric potential at the object surface but rather the vector of voltages induced by the total magnetic field in pickup coils.

[Literature on this: Griffiths H., Magnetic induction tomography. Meas. Sci. Technol. 26: 1126 - 1131. Korzhenevskii A. V., and V. A. Cherepenin. Magnetic induction tomography, J. Commun. Tech. Electron. 42; 469 - 474,1997].

The document WO 2008/011649 A1, which is considered as a representative state of the art refers to a method and a device for MIT, whereby a measurement is carried out using at least two different frequencies and an additional perturbation of the coils and/or the field geometry so as to determine a correction factor with which spurious signals generated by changes of the geometry and amplifier drift during the object measurement can be substantially eliminated.

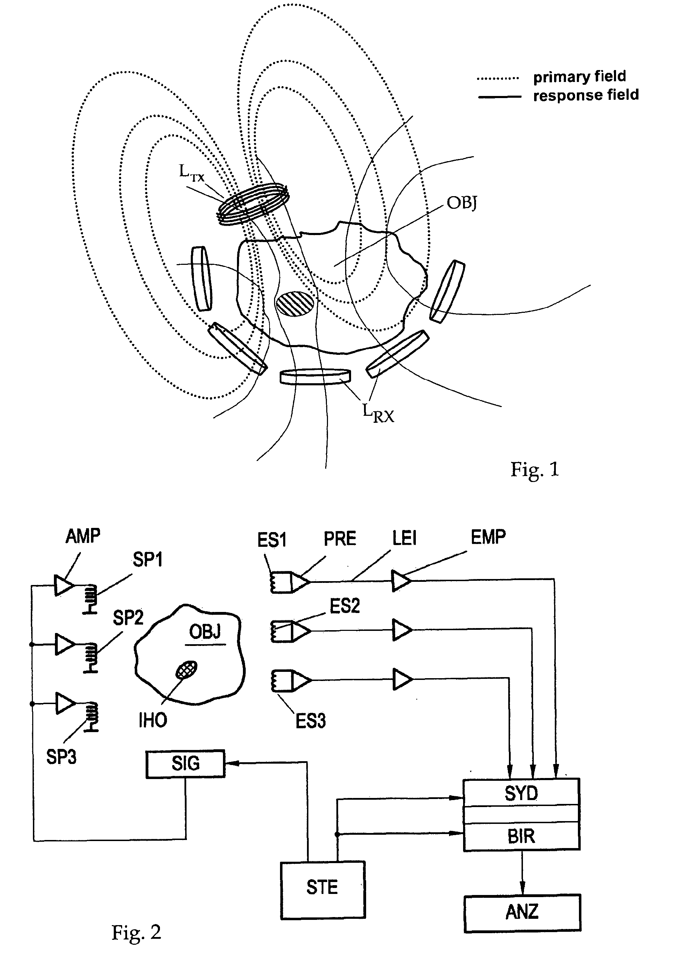

The principle of MIT is illustrated in Fig. 1 where the magnetic field is produced with a transmitter coil L_{TX} and coupled to the body OBJ under investigation. The resulting total field is sensed by an array of receiver coils L_{RX}.

Figure 2 shows schematically an object OBJ to be investigated, having an inhomogeneity IHO which has a conductivity different from the remainder of the object, for example, a lesion inside a part of the body such as the brain or a female breast.

Three transmitter coils SP1, SP2 and SP3 are arranged at various positions outside the object to be investigated, but as close as possible thereto, but the number of transmitter coils can also be substantially higher according to the desired resolution and the type of object. These transmitter coils are supplied with AC current, originating from a signal generator SIG, having amplifiers AMP connected ahead thereof for each transmitter coil. Three receiver coils ES1, ES2, ES3 are located in the area of the transmitter coils here but can also be arranged at completely different positions. A pre-amplifier PRE is provided for each of the receiver coils and these pre-amplifiers are connected via shielded lines LE1 to further amplifiers EMP whose outputs are supplied to a synchronous detector SYD. The synchronous detector SYD receives the necessary synchronous signal from the sine generator SIG. An image reconstruction BIR also takes place in the unit with the synchronous detector and its output signal can then be passed to a display ANZ such as a screen, a printer etc. The synchronous detector SYD, the amplifiers AMP and the image reconstruction BIR are controlled by a control unit STE. A coil designated as REF is used to obtain a reference signal.

Since the signals to be evaluated which are picked up by the receiver coils are in fact many orders of magnitude smaller than the excitation signals of the transmitter coils, care is initially taken to ensure that the fields of the transmitter coils do not act directly on the receiver coils. For this purpose, the receiver coils may be configured as so-called gradiometer coils which can additionally be arranged orthogonally in relation to the transmitter coils. Such gradiometer coils are in principle insensitive to other fields as long as these fields are homogeneous since the same voltage but with opposite sign is induced in each coil half. Since neither the receiver coil geometry is perfect nor are the interference fields which occur actually homogeneous, appreciable spurious signals occur however, partly e. g. from long- to short-wavelength transmitters. The processing by a synchronous detector in a known manner can considerably reduce the perturbation level here.

The signals received in the receiver coils ES1, ES2 and ES3 depend, inter alia, on the distribution of the electrical conductivity inside the object OBJ to be investigated and it has been shown that tissue variations in the breast tissue, for example, lead to conductivity variations which are sufficiently large to allow a mammographic representation following evaluation in a microprocessor of the image processing DVA. Details need not be discussed here since these can be found, for example, in the citation already mentioned.

A basic presentation on the multi-frequency modification MIT can be found in Hermann Scharfetter, Roberto Casanas and Javier Rosell, "Biological Tissue Characterization by Magnetic Induction Spectroscopy (MIS): Requirements and Limitations", IEEE Trans. Biomed. Eng. 50, 870 - 880, 2003.

In principle single-shot data acquisition can be achieved fairly easily, e. g. by applying the principle in "Scharfetter H., Köstinger A, Issa S. Hardware for quasi-single-shot multifrequency magnetic induction tomography (MIT): the Graz Mk2 system. Physiol. Meas. 2008; 29: S. 431 - S. 443", where all transmitter coils are powered at the same time. Different coils receive currents with slightly different frequencies, so that it is possible to separate their contributions in the receivers by synchronous demodulation. The spacing between the individual carriers is chosen so small that compared to the beta-dispersion of most tissues the carriers can be seen as practically one single frequency. One major problem is introduced by the driving amplifier of the transmitter coils: If its output impedance is low, each transmitter coil acts as if it were short circuited when seen from the receivers. This means that it perturbs the primary field considerably. This has the following consequences:
The field perturbation has to be considered in the reconstruction algorithm which requires the explicit modelling of the coils and sophisticates the numerical solvers.

All drifts of the electrical parameters in the termination impedance of the transmitter coils, i. e. the coil inductance and resistance as well as the output impedance of the power amplifiers become visible in the received signals. Such interferences can become very large compared to the object signal. Vibrational noise due to mutual coil movements can be enhanced significantly.

Magnetic coupling between the transmitter coils can propagate noise generated by the power amplifiers to the receiver coils to a wider extend than if the transmitter coils were magnetically decoupled.

This problem has only been tackled in the publication mentioned before so far, where the authors decided to drive the coils by current sources. However, the performance of current sources built with amplifiers and feedback networks gets poor when approaching high frequencies and driving currents due to stability problems.

### Summary of the invention

It is an object of the present invention to provide a high termination impedance to a transmitter coil and to drive the same coil at two or more different frequencies.

It is a further object of the invention to provide an alternative way to drive the transmitter coils which can be realized with simple passive networks and drivers which are configured as simple voltage sources without feedback loops.

This object is achieved by an apparatus according to the preamble of claim 1 which according to the invention is characterized by the fact that two or more feeding paths for the two or more frequencies are provided for each transmitter coil, in each feeding path the transmitter coil being driven at the corresponding frequency, via a λ/4-transformer, said coil being in series resonance with a series capacitor of said path at said one frequency, whereas the other feeding path(s) showing essentially high impedance for said one frequency.

In many cases it is advantageous if the λ/4-transformers are LC-II-networks, in order to avoid large dimensions.

In other cases the λ/4-transformers may be made from transmission lines, especially if the cable acts as the feeder line.

An embodiment which can realized for two frequencies in an easy way is **characterized in that** a low frequency path runs from the output of the power amplifier via the inductance of a first parallel resonance circuit, a low frequency λ/4-transformer, a series resonance capacitor for the transmitter coil at said low frequency, the transmitter coil and the inductance of a second parallel resonance circuit to ground, whereas a high frequency path runs from the output of the power amplifier via a high frequency λ/4-transformer , a series resonance capacitor for the transmitter coil at said high frequency, the transmitter coil, the series resonance capacitor for the transmitter coil at said low frequency and a capacitor of the low frequency λ/4-transformer to ground.

### Brief description of the drawings

The specific features and advantages of the present invention will become better understood with regard to the following description. In the following, the present invention is described in more detail with reference to the drawings, which show:
Fig. 1 illustratively and schematically the principle of MIT,
Fig. 2 in a block diagram the principle of a measurement arrangement in accordance with the state of the art,
Fig. 3 the transformation of the low output impedance of a power amplifier into a high termination impedance for a transmitter coil, avoiding a nearly purely reactive load for the power amplifier by using a series resonance capacitor,
Fig. 4 a complete dual frequency circuit,
Fig. 5 the circuit of Fig. 4, the low frequency path being marked,
Fig. 6 the circuit of Fig. 4, the high frequency path being marked,
Fig. 7 the termination impedance seen by the transmitter coil as a function of frequency in simulation test and
Fig. 8 the transmitter coil current as a function of frequency in simulation test.

### Detailed description of the invention

Usually two frequencies are used since it is known that the simultaneous use of two frequencies can enhance the contrast of the resulting pictures and helps to minimize artifacts e.g. due to object movements etc. If the spacing between the frequencies is large enough (e. g. 1 MHz - 10 MHz) a high termination impedance to the transmitter coil can be achieved by two λ/4 line transformers or the corresponding passive π-network made from lumped elements (L, C). Such networks transform a low impedance at one port into a high impedance at the second port according to Z₁= Z₀²/Z₂ where Z₁, Z₂ are the impedances at ports 1 and 2 and Zo is the characteristic impedance of the coupler. Therefore using a simple push-pull amplifier with low output impedance (e. g. CMOS-inverters which are also used in switching mode power supplies or in RFID-transmitters) can drive a coil through the transformer while the coil 'sees' a high impedance and thus does not provide a field perturbation.

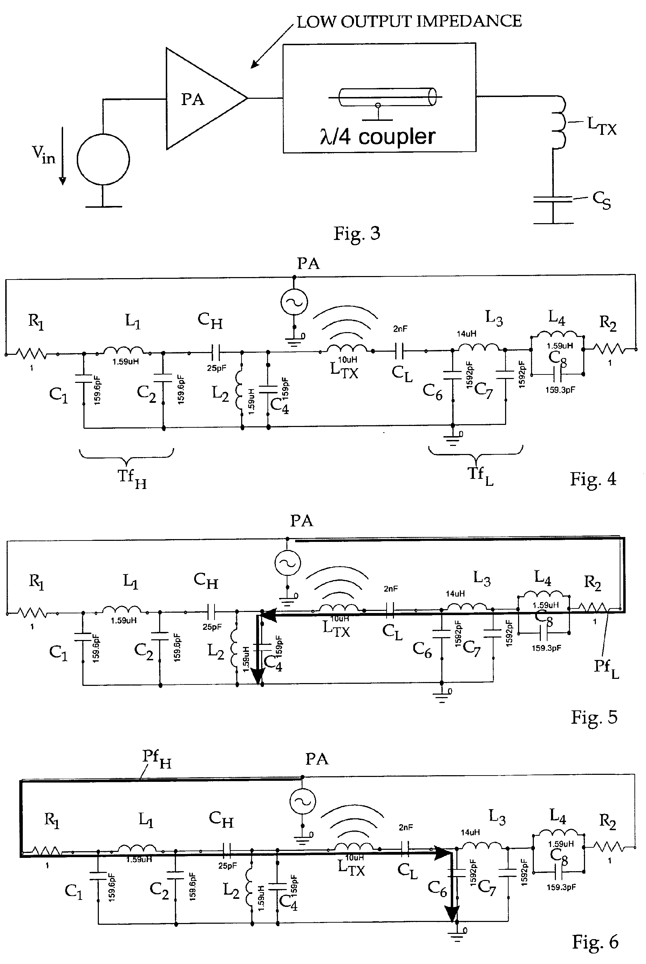

One major drawback of this concept is that the λ/4-coupler transforms the inductance of the transmitter coil L_{TX} in a capacitance so that the power amplifier sees a capacitive load which is usually very unfavorable for safe operation. It is therefore advisable to drive the coil L_{TX} in resonance by introducing a series capacitor C_{S}. This is shown in Fig. 3, where a power amplifier PA is driven by a voltage Vᵢₙ.

The goal to drive the same coil at two different frequencies can be achieved by providing two feeding paths for the same transmitter coil whereas one path provides a low frequency f_{L} path and the other one a high frequency f_{H} path. Fig. 4 shows such a network which has already been specified for f_{L} = 1 MHz and f_{H} = 10 MHz.

Here a low frequency path Pf_{L} runs from the output of the power amplifier PA via the inductance L₄ of a first parallel resonance circuit L₄, C₈, a low frequency λ/4-transformer Tf_{L}, a series resonance capacitor C_{L} for the transmitter coil L_{RX} at said low frequency f_{L}, the transmitter coil and the inductance L₂ of a second parallel resonance circuit L₂, C₄ to ground, whereas a high frequency path Pf_{H} runs from the output of the power amplifier PA via a high frequency λ/4-transformer Tf_{H}, a series resonance capacitor C_{H} for the transmitter coil L_{RX} at said high frequency f_{H}, the transmitter coil, the series resonance capacitor C_{L} for the transmitter coil at said low frequency f_{L} and a capacitor C₆ of the low frequency λ/4-transformer Tf_{L} to ground.

The low frequency λ/4-transformer Tf_{L} is represented by a II-circuit, consisting of C₆, C₇ and L₃, whereas the high frequency λ/4-transformer Tf_{H} is represented by a II-circuit, consisting of C₁, C₂ and L₁.

The frequencies of the two parallel resonance circuit L₄, C₈ and L₂, C₄ are principally the high frequency f_{H} (10 MHz) in the example described. However it should me mentioned that in the shown example the high frequency λ/4-transformer Tf_{H} does not provide an infinitely high impedance at the junction C₂/C_{H} due to the nonzero input impedance R₁. Thus C_{H} contributes, though very little, to the overall resonance frequency of the circuit L₂, C₄. Therefore the resonance frequency of circuit L₂, C₄ in this case is 10,0097 MHz.

As can be seen two feeding resistors R1, R2 are provided, here representing the output resistance of the power amplifier PA for simulation purposes only. These resistors are not required for a proper functioning of the circuit and will thus be omitted in the real design.

However, as this circuit may be difficult to understand due to its complexity, the operation is illustrated hereunder in connection with Figs. 5 and 6. Fig 5 shows the LF feeding path Pf_{L}. The LF signal with the frequency f_{L} travels through the inductance L₄ of the HF blocking filter L₄/C₈, the LF-coupler Tf_{L}, the LF-series resonator formed by the transmitter coil L_{RX} and C_{L} and the inductance L₂ of the HF blocking filter, the parallel resonance circuit L₂/C₄. The HF feeding network is blocked by the small capacitance C_{H} which provides a high impedance at the low frequency f_{L}.

The termination of the transmitter coil L_{RX} at the low frequency f_{L}: No closed low-impedance current path is provided because the LF-λ/4 transformer shows high impedance and the HF resonance capacitor C_{H} has high impedance at LF. Any current path is interrupted so that the transmitter coil L_{RX} actually sees a high termination impedance.

The analysis of the HF path Pf_{H} can be done in an analogous manner. Fig. 6 shows the HF feeding path. Here the series resonator is made up by L_{TX} and the three capacitors C_{H} and C_{L} and C₆. However, as C_{H} is comparatively small, the resonance frequency is essentially determined by C_{H}. C₆ provides a convenient path to ground while the HF- λ/4 transformer Tf_{H} establishes the connection to the power amplifier PA.

The termination of the transmitter coil at the high frequency f_{H}:. No closed low-impedance current path is provided because the HF-λ/4 transformer Tf_{H} shows high impedance and the network formed by L₂ and C₄ represents a parallel resonator at HF. Possible current paths are interrupted so that the transmitter coil L_{TX} again sees a high termination impedance.

Needless to say that the impedance transformers can also be made from transmission lines, but then the length may become impractically large and the characteristic impedance cannot be chosen arbitrarily when relying on commercially available cables (50 Ω and 75 Ω). For 10 MHz e. g. the cable length of a commercial 50 Ω line must be approx. 5 m. This is still feasible, especially if the cable simultaneously acts as the feeder line. At lower frequencies a circuit with lumped elements is certainly preferable. Transmission line couplers, however, have the nice property that they operate at all odd multiples of the quarter wavelength and hence are intrinsically suitable for certain multifrequency applications where such multiples can be used as carriers.

The expert may use other types and combinations of crossover networks, even if more than two frequencies are to be applied.

In the context of carrier frequency splitting as mentioned above in the introduction the bandwidth of the resonators must be chosen such that it covers the full span of the individual carrier frequencies for the different transmitter coils.

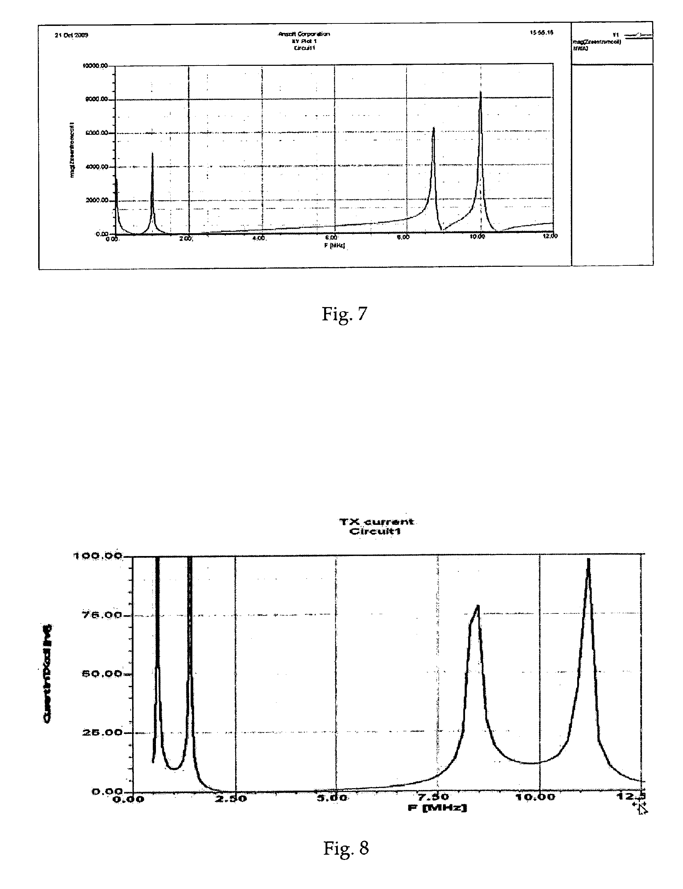

Fig. 7 shows the termination impedance as seen from the transmitter coil L_{TX} when simulating the network presented in Fig 4 assuming a driver output impedance of 1 Ω. Simulations were done with Ansoft Designer SV, ver 2.2.0 (evaluation version, Ansoft Corp.). The impedance at the low frequency f_{L} = 1 MHz is 5.5 kΩ, at the high frequency f_{H} = 10 MHz a value of > 8 kΩ is reached. The values depend on both the driver output impedance and the characteristic impedance Zo of the λ/4 coupler. Higher Zo yields higher termination impedance but at the same time reduces the coil current at constant driver voltage. Thus higher impedances can only be reached when simultaneously increasing the driving voltage.

Fig. 8 shows the current in the transmitter coil L_{TX} when the power amplifier PA drives the circuit with a voltage of 1 V. As can be seen there are approx. 10 mA being delivered at both target frequencies. As driving voltages of 10 V are easily achievable, driving 100 mA is no problem.

## Claims

1. Apparatus for magnetic induction tomography comprising
at least one transmitter coil (LTX) for the introduction of an alternating magnetic field into a target body (OBJ) to be investigated,
at least one receiver coil (LRX) for the pick-up of received signals and
means (STE, SYD, BIR) for processing the received signals which reconstructs an image of the spatial electrical properties in the object from the amplitudes and phases of the received signals,
the at least one transmitter coil being driven by a power amplifier (PA) having a low output impedance and working at two or more different frequencies (f_{L}, f_{H}),
**characterized in that**
two or more feeding paths (Pf_{L}, Pf_{H}) for the two or more frequencies (f_{L}, f_{H}) are provided for each transmitter coil (L_{TX}),
in each feeding path the transmitter coil being driven at the corresponding frequency (f_{L}, f_{H}), via a λ/4-transformer (Tf_{L}, Tf_{H}), said coil being in series resonance with a series capacitor (C_{L}, C_{H}) of said path at said one frequency, whereas the other feeding path(s) showing essentially high impedance for said one frequency.

2. Apparatus according to claim 1, **characterized in that** the λ/4-transformers are LC-II-networks (C₆, C₇, L₃; C₁, C₂, L₁)

3. Apparatus according to claim 1, **characterized in that** the λ/4-transformers are made from transmission lines.

4. Apparatus according to claim 1, **characterized in that** a low frequency path (Pf_{L}) runs from the output of the power amplifier (PA) via the inductance (L₄) of a first parallel resonance circuit (L₄, C₈), a low frequency λ/4-transformer (Tf_{L}), a series resonance capacitor (C_{L}) for the transmitter coil at said low frequency (f_{L}), the transmitter coil and the inductance (L₂) of a second parallel resonance circuit (L₂, C₄) to ground,
whereas a high frequency path (Pf_{H}) runs from the output of the power amplifier (PA) via a high frequency λ/4-transformer (Tf_{H}), a series resonance capacitor (C_{H}) for the transmitter coil at said high frequency (f_{H}), the transmitter coil, the series resonance capacitor (C_{L}) for the transmitter coil at said low frequency (f_{L}) and a capacitor (C₆) of the low frequency λ/4-transformer (Tf_{L}) to ground.
